# EUROPÄISCHE PATENTSCHRIFT

(11) **EP 0 615 521 B1**
(45) Veröffentlichungstag und Bekanntmachung des Hinweises auf die Patenterteilung: **05.03.1997**
(21) Anmeldenummer: 92922591.0
(22) Anmeldetag: 30.10.1992
(51) Int. Cl.: C07H 15/04, A61K 7/08, A61K 7/48

(54) **ALKYL- UND/ODER ALKENYLOLIGOGLYKOSID-ISETHIONATE**
ALKYL AND/OR ALKENYL OLIGOGLYCOSIDE ISETHIONATES
ALKYL- ET/OU ALKENYLOLIGOGLUCOSIDE-ISETHIONATES

(30) Priorität: 08.11.1991 DE 4136783
(43) Veröffentlichungstag der Anmeldung: 21.09.1994
(73) Patentinhaber: Henkel Kommanditgesellschaft auf Aktien, 40191 Düsseldorf (DE)
(72) Erfinder: WEUTHEN, Manfred, D-5650 Solingen 11 (DE)
(86) Internationale Anmeldenummer: EP9202500
(87) Internationale Veröffentlichungsnummer: WO9309125

(56) Entgegenhaltungen:
- EP-A- 0 301 298
- EP-A- 0 362 671
- US-A- 2 580 352
- US-A- 4 990 609
- Chemical Abstracts, Band 116, Nr. 23, 8 Juni 1992, (Columbus, Ohio, US),
- Zusammenfassung 236083u, JP, A2, 3291295, (Yamamuro Akira et al.) 20 Dezember 1991

## Beschreibung

Die Erfindung betrifft Alkyl- und/oder Alkenyloligoglykosid-isethionate, erhältlich durch Umsetzung von Alkyl- und/oder Alkenyloligoglykosiden mit Vinylsulfonaten in Gegenwart basischer Verbindungen, ein Verfahren zu ihrer Herstellung sowie ihre Verwendung in oberflächenaktiven Mitteln.

### Stand der Technik

Isethionate stellen Aniontenside mit einer -O-CH₂CH₂-SO₃⁻- Gruppe dar, die infolge ihrer guten dermatologischen Verträglichkeit bevorzugt in kosmetischen Mitteln Verwendung finden [**Parf.Kosm., 42, 203 (1961)**]. Zu ihrer Herstellung geht man gewöhnlich von Fettsäuren oder deren Estern aus, die mit Natriumisethionat, Propan- oder Butansulton kondensiert werden [**Bull.Chem.Soc.Jap., 43, 2236 (1970)**].

Von Nachteil ist hierbei jedoch, daß der Umgang mit Isethionsäuresalzen und Sultonen hohe Anforderungen an die Arbeitssicherheit stellt und die Produkte geringe Mengen dieser Stoffe als Verunreinigungen enthalten können, was aus toxikologischer Sicht unerwünscht ist. Isethionate nach dem Stand der Technik sind ferner nicht alkalistabil und können Fettsäuren als Hydrolyseprodukte enthalten, was ihre anwendungstechnischen Eigenschaften nachteilig beeinflußt.

Die Aufgabe der Erfindung bestand somit darin, neue Isethionate zu entwickeln, die frei von den geschilderten Nachteilen sind.

### Beschreibung der Erfindung

Gegenstand der Erfindung sind Alkyl- und/oder Alkenyloligoglykosid-isethionate, die man erhält, in dem man Alkyl- und/ oder Alkenyloligoglykoside der Formel (I)

R¹O-[G]ₚ (I)

in der
- R¹: für einen aliphatischen, linearen oder verzweigten Kohlenwasserstoffrest mit 6 bis 22 Kohlenstoffatomen und 0, 1, 2 oder 3 Doppelbindungen,
- [G]: für eine Glykose-Einheit mit 5 oder 6 Kohlenstoffatomen und
- p: für eine Zahl zwischen 1 und 10 steht,
in Gegenwart von basischen Verbindungen bei erhöhter Temperatur mit Salzen der Vinylsulfonsäure umsetzt.

Überraschenderweise wurde gefunden, daß Alkyl- und/oder Alkenyloligoglykoside in Gegenwart basischer Katalysatoren mit Vinylsulfonaten zu Verbindungen mit Isethionatstruktur umgesetzt werden können. Die Produkte zeigen eine unerwartet gute dermatologische Verträglichkeit, ausgezeichnete Detergenseigenschaften, hohe Alkalistabilität und sind biologisch leicht abbaubar.

Alkyloligoglykosid-isethionate mit besonders vorteilhaften anwendungstechnischen Eigenschaften werden erhalten, wenn man von Alkyloligoglykosiden der Formel (I) ausgeht, in der R¹ für einen Alkylrest mit 12 bis 18 Kohlenstoffatomen, G für einen Glucoserest und/oder p für Zahlen von 1,1 bis 3,0 steht.

Ein weiterer Gegenstand der Erfindung betrifft ein Verfahren zur Herstellung von Alkyl- und/oder Alkenyloligoglykosid-isethionaten, das sich dadurch auszeichnet, daß man Alkyl- und/oder Alkenyloligoglykoside der Formel (I)

R¹O-[G]ₚ (I)

in der
- R¹: für einen aliphatischen, linearen oder verzweigten Kohlenwasserstoffrest mit 6 bis 22 Kohlenstoffatomen und 0, 1, 2 oder 3 Doppelbindungen,
- [G]: für eine Glykose-Einheit mit 5 oder 6 Kohlenstoffatomen und
- p: für eine Zahl zwischen 1 und 10 steht,
in Gegenwart von basischen Verbindungen bei erhöhter Temperatur mit Salzen der Vinylsulfonsäure umsetzt.

**Alkyl- und/oder Alkenylglykoside** stellen bekannte Stoffe dar, die nach den einschlägigen Methoden der präparativen organischen Chemie zugänglich sind. Zu ihrer Herstellung kann man von Mono- oder Polyzuckern, wie beispielsweise Glucose oder Stärke ausgehen, die in Gegenwart saurer Katalysatoren entweder direkt oder über die Zwischenstufe niederer Alkyl- und/ oder Alkenylglykoside mit Fettalkoholen acetalisiert werden. Stellvertretend für das umfangreiche Schrifttum zu diesem Thema sei hier auf die Europäischen Patentanmeldungen **EP 0 301 298 A1** und **EP 0 362 671 A1** verwiesen.

Zur Herstellung der erfindungsgemäßen Alkyl- und/oder Alkenylglykosid-isethionate kommen als Ausgangsstoffe Alkyl- und/oder Alkenylglykoside der Formel (I) in Betracht, bei denen sich die Glycose-Einheit [G] von Aldosen bzw. Ketosen ableitet. Vorzugsweise werden wegen der besseren Reaktionsfähigkeit die reduzierend wirkenden Saccharide, die Aldosen, verwendet. Unter den Aldosen kommt wegen ihrer leichten Zugänglichkeit und technischen Verfügbarkeit insbesondere die Glucose in Betracht. Die als Ausgangsstoffe besonders bevorzugt eingesetzten Alkyl- und/oder Alkenylglykoside sind daher die Alkyl- und/oder Alkenyl**glucoside.**

Die Indexzahl p in der allgemeinen Formel (I) gibt den Oligomerisierungsgrad, d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkylglykosid eine analytisch ermittelte rechnerische Größe, die meistens eine gebrochene Zahl darstellt. Vorzugsweise werden Alkyl- und/oder Alkenylglykoside mit einem mittleren Oligomerisierungsgrad p von 1,1 bis 3,0 verwendet. Besonders bevorzugt sind solche Alkyl- und/oder Alkenylglykoside, deren Oligomerisierungsgrad kleiner als 1,7 ist und insbesondere zwischen 1,2 und 1,4 liegt.

Der Alkylrest R¹ kann sich von primären Alkoholen mit 6 bis 22, vorzugsweise 12 bis 18 Kohlenstoffatomen ableiten. Typische Beispiele sind Capronalkohol, Caprylalkohol, Caprinalkohol, Laurylalkohol, Myristylalkohol, Cetylalkohol, Palmoleylalkohol, Stearylalkohol, Oleylalkohol, Elaidylalkohol, Petroselinylalkohol, Linolylalkohol, Linolenylalkohol, Arachylalkohol, Gadolylalkohol, Behenylalkohol, Erucylalkohol sowie deren technische Mischungen auf Basis von natürlichen Fetten und Ölen, beispielsweise Palmöl, Palmkernöl, Kokosöl oder Rindertalg.

Bevorzugt ist der Einsatz von Alkyloligoglykosiden der Formel (I), in der R¹ für einen Alkylrest mit 12 bis 18 Kohlenstoffatomen, [G] für einen Glucoserest und/oder p für Zahlen von 1,1 bis 3,0 steht.

Unter den **Salzen der Vinylsulfonsäure** sind die Alkali- und/ oder Erdalkalisalze der Ethylensulfonsäure (CH₂=CH-SO₃H) zu verstehen. Typische Beispiele sind die Lithium-, Kalium-, Calcium- und Magnesiumsalze der Vinylsulfonsäure. Bevorzugt ist der Einsatz von Vinylsulfonsäure-Natriumsalz.

Je nachdem, wieviele der in den Alkyl- und/oder Alkenyloligoglykosiden vorhandenen freien Hydroxylgruppen verethert werden sollen, kann das molare Verhältnis von Alkyl- und/oder Alkenyloligoglykosid zu Vinylsulfonsäure-Salz 1 : 5 bis 10 : 1 betragen. Sowohl aus wirtschaftlicher Sicht, als auch im Hinblick auf die Eigenschaften der Isethionate hat es sich als optimal erwiesen, die Reaktionspartner im molaren Verhältnis von 3 : 1 bis 1 : 2 einzusetzen.

Unter **basischen Verbindungen** sind im Sinne der Erfindung die Oxide, Hydroxide, Carbonate und/oder C₁-C₄-Alkoholate der Alkali- und/oder Erdalkalimetalle zu verstehen. Typische Beispiele sind Lithiumhydroxid, Natriumhydroxid, Kaliumhydroxid, Calciumoxid, Calciumhydroxid, Magnesiumoxid, Magnesiumhydroxid, Natriumcarbonat, Magnesiumcarbonat, Natriummethylat, Natriumethylat und Kalium-tert.butylat. Bevorzugt ist die Verwendung von Kaliumcarbonat.

Die basischen Verbindungen können dabei in Mengen von 0,1 bis 10, vorzugsweise 0,5 bis 1,5 Gew.-% - bezogen auf die Alkyl- und/oder Alkenyloligoglykoside - eingesetzt werden.

Zur Durchführung der Reaktion zwischen den Alkyl- und/oder Alkenyloligoglykosiden und den Vinylsulfonaten sind erhöhte Temperaturen von 50 bis 200°C erforderlich.

In einer besonderen Ausführungsform des erfindungsgemäßen Verfahrens können die Reaktionspartner in Substanz - also wasserfrei - miteinander umgesetzt werden. In diesem Fall sind Reaktionstemperaturen oberhalb des Schmelzpunktes der Alkyl- und/oder Alkenylglykoside von mindestens 120°C erforderlich. In einer weiteren Ausführungsform können die Glykoside und die Vinylsulfonate in wäßriger Lösung zur Reaktion gebracht werden. Dies kann unter deutlich milderen Beding- ungen von 50 bis 95°C erfolgen. Die Reaktionszeit beträgt in beiden Fällen 1 bis 24, vorzugsweise 5 bis 12 h und wird maßgeblich durch die Katalysatorkonzentration beeinflußt.

Die Reaktion zwischen Alkyl- und Alkyloligoglykosiden und Vinylsulfonaten erfolgt zwischen einer Hydroxylgruppe des Glykosids und der Doppelbindung des Vinylsulfonsäure-Salzes. In Abhängigkeit von der Menge an eingesetztem Vinylsulfonat können sowohl Mono- als auch Oligoether des Glykosids gebildet werden, die als statistisches Gemisch anfallen.

Alkyl- und/oder Alkenyloligoglykosid-isethionate zeigen ausgezeichnete Detergenseigenschaften und eignen sich daher zum Einsatz in oberflächenaktiven Mitteln.

Ein weiterer Gegenstand der Erfindung betrifft daher die Verwendung von Alkyl- und/oder Alkenyloligoglykosid-isethionaten zur Herstellung von Wasch-, Spül- und Reinigungsmitteln sowie Produkten der Haar- und Körperpflege, in denen sie in Mengen von 0,1 bis 25, vorzugsweise 1 bis 10 Gew.-% - bezogen auf die Mittel - enthalten sein können.

Die folgenden Beispiele sollen den Gegenstand der Erfindung näher erläutern, ohne ihn darauf einzuschränken.

### Beispiele

### Beispiel 1:

In einem 500-ml-Dreihalskolben mit Rührer, Innenthermometer und Tropftrichter wurden 250 g (1 mol) Lauryloligoglucosid (Hydroxylzahl: 656, durchschnittlicher Oligomerisierungsrad 1,3, Fa.Henkel KGaA, Düsseldorf, FRG) und 2,8 g (0,02 mol) Kaliumcarbonat vorgelegt und auf 160°C erhitzt. Anschließend wurde die Mischung über einen Zeitraum von 1 h portionsweise mit 30 g (0,23 mol) Vinylsulfonsäure-Natriumsalz versetzt, wobei die Reaktionstemperatur allmählich auf 190°C gesteigert wurde. Nach weiteren 3 h wurde die Reaktion abgebrochen, das feste Reaktionsprodukt zerkleinert und in Wasser dispergiert.

### Beispiel 2:

In einem 500-ml-Dreihalskolben mit Rührer, Innenthermometer und Tropftrichter wurden 170 g (0,7 mol) Lauryloligoglucosid aus Beispiel 1 und 4,5 g (0,03 mol) Kaliumcarbonat vorgelegt und bei einer Temperatur von 90°C in ca. 50 ml Wasser gelöst. Über einen Zeitraum von 1 h wurde die Reaktionsmischung portionsweise mit 26 g (0,2 mol) Vinylsulfonsäure-Natriumsalz in Form einer 30 gew.-%igen wäßrigen Lösung versetzt und weitere 8 h gerührt.

## Patentansprüche

1. Alkyl- und/oder Alkenyloligoglykosid-isethionate, dadurch erhältlich, daß man Alkyl- und/oder Alkenyloligoglykoside der Formel (I)
R¹O-[G]ₚ (I)
in der
R¹ für einen aliphatischen, linearen oder verzweigten Kohlenwasserstoffrest mit 6 bis 22 Kohlenstoffatomen und 0, 1, 2 oder 3 Doppelbindungen,
[G] für eine Glykose-Einheit mit 5 oder 6 Kohlenstoffatomen und
p für eine Zahl zwischen 1 und 10 steht,
in Gegenwart von basischen Verbindungen bei erhöhter Temperatur mit Salzen der Vinylsulfonsäure umsetzt.

2. Alkyl- und/oder Alkenyloligoglykosid-isethionate nach Anspruch 1, **dadurch gekennzeichnet,** daß in der Formel (I) R¹ für einen Alkylrest mit 12 bis 18 Kohlenstoffatomen steht.

3. Alkyl- und/oder Alkenyloligoglykosid-isethionate nach den Ansprüchen 1 und 2, **dadurch gekennzeichnet,** daß in der Formel (I) [G] für einen Glucoserest steht.

4. Alkyl- und/oder Alkenyloligoglykosid-isethionate nach mindestens einem der Ansprüche 1 bis 3, **dadurch** **gekennzeichnet,** daß in der Formel (I) p für Zahlen von 1,1 bis 3,0 steht.

5. Verfahren zur Herstellung von Alkyl- und/oder Alkenyloligoglykosid-isethionaten, **dadurch gekennzeichnet,** daß man Alkyl- und/oder Alkenyloligoglykoside der Formel (I)
R¹O-[G]ₚ (I)
in der
R¹ für einen aliphatischen, linearen oder verzweigten Kohlenwasserstoffrest mit 6 bis 22 Kohlenstoffatomen und 0, 1, 2 oder 3 Doppelbindungen,
[G] für eine Glykose-Einheit mit 5 oder 6 Kohlenstoffatomen und
p für eine Zahl zwischen 1 und 10 steht,
in Gegenwart von basischen Verbindungen bei erhöhter Temperatur mit Salzen der Vinylsulfonsäure umsetzt.

6. Verfahren nach Anspruch 5, **dadurch gekennzeichnet,** daß man Alkyl- und/oder Alkenyloligoglykoside der Formel (I) einsetzt, in der R¹ für einen Alkylrest mit 12 bis 18 Kohlenstoffatomen steht.

7. Verfahren nach den Ansprüchen 5 und 6, **dadurch gekennzeichnet,** daß man Alkyl- und/oder Alkenyloligoglykoside der Formel (I) einsetzt, in der [G] für einen Glucoserest steht.

8. Verfahren nach mindestens einem der Ansprüche 5 bis 7, **dadurch gekennzeichnet**, daß man Alkyl- und/oder Alkenyloligoglykoside der Formel (I) einsetzt, in der p für Zahlen von 1,1 bis 3,0 steht.

9. Verfahren nach mindestens einem der Ansprüche 5 bis 8, **dadurch gekennzeichnet,** daß man die Alkyl- und/oder Alkenyloligoglykoside und die Vinylsulfonsäure-Salze im molaren Verhältnis von 1 : 5 bis 10 : 1 einsetzt.

10. Verfahren nach mindestens einem der Ansprüche 5 bis 9, **dadurch gekennzeichnet,** daß man als basische Verbindungen Oxide, Hydroxide, Carbonate und/oder C₁-C₄-Alkoholate von Alkali- und/oder Erdalkalimetallen einsetzt.

11. Verfahren nach mindestens einem der Ansprüche 5 bis 10, **dadurch gekennzeichnet,** daß man die basischen Verbindungen in Mengen von 0,1 bis 10 Gew.-% - bezogen auf die Alkyl- und/oder Alkenyloligoglykoside - einsetzt.

12. Verfahren nach mindestens einem der Ansprüche 5 bis 11, **dadurch gekennzeichnet,** daß man die Reaktion bei Temperaturen von 50 bis 200°C durchführt.

13. Verwendung der Alkyl- und/oder Alkenyloligoglykosid-isethionate nach den Ansprüchen 1 bis 4 zur Herstellung von Wasch-, Spül- und Reinigungsmitteln sowie von Produkten zur Haar- und Körperpflege.

14. Verwendung der Alkyl- und/oder Alkenyloligoglykosid-isethionate nach dem Verfahren nach den Ansprüchen 5 bis 12 zur Herstellung von Wasch-, Spül- und Reinigungsmitteln sowie von Produkten zur Haar- und Körperpflege.

## Claims

1. Alkyl and/or alkenyl oligoglycoside isethionates obtainable by reaction of alkyl and/or alkenyl oligoglycosides corresponding to formula (I):
R¹O-[G]ₚ (I)
in which
R¹ is an aliphatic, linear or branched hydrocarbon radical containing 6 to 22 carbon atoms and 0, 1, 2 or 3 double bonds,
[G] is a glycose unit containing 5 or 6 carbon atoms and
p is a number of 1 to 10,
with salts of vinyl sulfonic acid at elevated temperature in the presence of basic compounds.

2. Alkyl and/or alkenyl oligoglycoside isethionates as claimed in claim 1, characterized in that, in formula (I) R¹ is an alkyl radical containing 12 to 18 carbon atoms.

3. Alkyl and/or alkenyl oligoglycoside isethionates as claimed in claims 1 and 2, characterized in that, in formula (I) [G] is a glucose unit.

4. Alkyl and/or alkenyl oligoglycoside isethionates as claimed in at least one of claims 1 to 3, characterized in that, in formula (I), p is a number of 1.1 to 3.0.

5. A process for the production of alkyl and/or alkenyl oligoglycoside isethionates, characterized in that alkyl and/or alkenyl oligoglycosides corresponding to formula (I):
R¹O-[G]ₚ (I)
in which
R¹ is an aliphatic, linear or branched hydrocarbon radical containing 6 to 22 carbon atoms and 0, 1, 2 or 3 double bonds,
[G] is a glycose unit containing 5 or 6 carbon atoms and
p is a number of 1 to 10,
are reacted with salts of vinyl sulfonic acid at elevated temperature in the presence of basic compounds.

6. A process as claimed in claim 5, characterized in that alkyl and/or alkenyl oligoglycosides corresponding to formula (I), in which R¹ is a C₁₂₋₁₈ alkyl radical, are used.

7. A process as claimed in claims 5 and 6, characterized in that alkyl and/or alkenyl oligoglycosides corresponding to formula (I), in which [G] is a glucose unit, are used.

8. A process as claimed in at least one of claims 5 to 7, characterized in that alkyl and/or alkenyl oligoglycosides corresponding to formula (I), in which p is a number of 1.1 to 3.0, are used.

9. A process as claimed in at least one of claims 5 to 8, characterized in that the alkyl and/or alkenyl oligoglycosides and the vinyl sulfonic acid salts are used in a molar ratio of 1:5 to 10:1.

10. A process as claimed in at least one of claims 5 to 9, characterized in that oxides, hydroxides, carbonates and/or C₁₋₄ alcoholates of alkali metals and/or alkaline earth metals are used as the basic compounds.

11. A process as claimed in at least one of claims 5 to 10, characterized in that the basic compounds are used in quantities of 0.1 to 10% by weight, based on the alkyl and/or alkenyl oligoglycosides.

12. A process as claimed in at least one of claims 5 to 11, characterized in that the reaction is carried out at temperatures of 50 to 200°C.

13. The use of the alkyl and/or alkenyl oligoglycoside isethionates as claimed in claims 1 to 4 for the production of laundry detergents, dishwashing detergents and cleaning products and for the production of hair-care and body-care formulations.

14. The use of the alkyl and/or alkenyl oligoglycoside isethionates obtained by the process claimed in claims 5 to 12 for the production of laundry detergents, dishwashing detergents and cleaning products and for the production of hair-care and body-care formulations.

## Revendications

1. Iséthionates d'alkyl- et/ou d'alcényloligoglycosides, obtenables en faisant réagir des alkyl- et/ou des alcényloligoglycosides de la formule (I)
R¹O-[G]ₚ (I)
dans laquelle
R¹ représente un radical d'hydrocarbure aliphatique, linéaire ou ramifié comportant 6 à 22 atomes de carbone et 0, 1, 2 ou 3 doubles liaisons,
[G] correspond à une unité d'ose comportant 5 ou 6 unités d'hydrocarbure et
p équivaut à un nombre entre 1 et 10,
avec des sels de l'acide vinylsulfonique, en présence de composés basiques et à température accrue.

2. Iséthionates d'alkyl- et/ou d'alcényloligoglycosides selon la revendication 1, **caractérisés en ce que**, dans la formule (I), R¹ représente un radical alkyle comportant 12 à 18 atomes de carbone.

3. Iséthionates d'alkyl- et/ou d'alcényloligoglycosides selon les revendications 1 et 2, **caractérisés en ce que**, dans la formule I, [G] correspond à un radical glucose.

4. Iséthionates d'alkyl- et/ou d'alcényloligoglycosides selon au moins une des revendications 1 à 3, **caractérisés en ce que**, dans la formule I, (p) équivaut à un nombre de 1,1 à 3,0.

5. Procédé de préparation d'iséthionates d'alkyl- et/ou d'alcényloligoglycosides, **caractérisé en ce que** l'on fait réagir des alkyl- et/ou des alcényloligoglycosides de la formule (I)
R¹O-[G]ₚ (I)
dans laquelle
R¹ représente un radical d'hydrocarbure aliphatique, linéaire ou ramifié comportant 6 à 22 atomes de carbone et 0, 1, 2 ou 3 doubles liaisons,
[G] correspond à une unité d'ose comportant 5 ou 6 unités d'hydrocarbure et
p équivaut à un nombre entre 1 et 10,
avec des sels de l'acide vinylsulfonique, en présence de composés basiques et à température accrue.

6. Procédé selon la revendication 5, **caractérisé en ce que** l'on utilise des alkyl- et/ou des alcényloligoglycosides de la formule (I) dans laquelle R¹ représente un radical alkyle comportant 12 à 18 atomes de carbone.

7. Procédé selon les revendications 5 et 6, **caractérisé en ce que** l'on utilise des alkyl- et/ou des alcényloligoglycosides de la formule (I) dans laquelle [G] correspond à un radical glucose.

8. Procédé selon au moins une des revendications 5 à 7, **caractérisé en ce que** l'on utilise des iséthionates d'alkyl- et/ou d'alcényloligoglycosides de la formule (I), dans laquelle p équivaut à un nombre de 1,1 à 3,0.

9. Procédé selon au moins une des revendications 5 à 8, **caractérisé en ce que** l'on utilise les alkyl- et/ou les alcényloligoglycosides et les sels de l'acide vinylsulfonique dans un rapport molaire compris de 1:5 à 10:1.

10. Procédé selon au moins une des revendications 5 à 9, **caractérisé en ce que** l'on utilise comme composés basiques, des oxydes, des hydroxydes, des carbonates et/ou des alcoolates en C₁-C₄ de métaux alcalins et/ou alcalino-terreux.

11. Procédé selon au moins une des revendications 5 à 10, **caractérisé en ce que** l'on met en oeuvre les composés basiques en quantités de 0,1 à 10 % en poids, par rapport aux alkyl- et/ou aux alcényloligoglycosides.

12. Procédé selon au moins une des revendications 5 à 11, **caractérisé en ce que** l'on opère la réaction à des températures de 50 à 200 °C.

13. Utilisation des iséthionates d'alkyl- et/ou d'alcényloligoglycosides selon les revendications 1 à 4, pour la fabrication de produits de lavage, de rinçage et de nettoyage ainsi que de produits pour le soin des cheveux et du corps.

14. Utilisation des iséthionates d'alkyl- et/ou d'alcényloligoglycosides selon le procédé décrit dans les revendications 5 à 12, pour la fabrication de produits de lavage, de rinçage et de nettoyage, ainsi que de produits pour le soin des cheveux et du corps.
